# EUROPEAN PATENT SPECIFICATION

(11) **EP 1 541 168 B1**
(45) Date of publication and mention of the grant of the patent: **24.11.2010**
(21) Application number: 04106364.5
(22) Date of filing: 07.12.2004
(51) Int. Cl.: A61K 31/57, A61K 38/24, A61P 15/08, A61K 31/5575, A61D 19/00

(54) **Method of synchronising ovulation in cattle**
Methode zur Synchronisierung des Eisprungs bei Rindern
Methode de synchronisation de l'ovulation chez le bétail

(30) Priority: 09.12.2003 EP 03104611
(43) Date of publication of application: 15.06.2005
(73) Proprietor: Intervet International BV, 5831 AN Boxmeer (NL)
(72) Inventor: Driancourt, Marc-Antoine, 49330 Chateauneuf/Sarthe (FR)
(74) Representative: Stumm, Karin

(56) References cited:
- WO-A-02/12441
- RICHARDSON A M ET AL: "Characteristics of estrus before and after first insemination and fertility of heifers after synchronized estrus using GnRH, PGF2alpha, and progesterone." JOURNAL OF ANIMAL SCIENCE, vol. 80, no. 11, November 2002 (2002-11), pages 2792-2800, XP002291498 ISSN: 0021-8812
- STEVENSON J S ET AL: "Synchronizing estrus and(or) ovulation in beef cows after combinations of GnRH, norgestomet, and prostaglandin F2alpha with or without timed insemination" JOURNAL OF ANIMAL SCIENCE, vol. 78, no. 7, July 2000 (2000-07), pages 1747-1758, XP002291499 ISSN: 0021-8812
- RAO A V ET AL: "ESTROUS RESPONSE AND FERTILITY IN POSTPARTUM ANESTROUS BUFFALOS TREATED WITH A PROGESTAGEN, PREGNANT MARE SERUM GONADOTROPIN AND PROSTAGLANDIN DURING THE LOW BREEDING SEASON" CHEMABS, 1985, XP002167862
- NEGLIA GIANLUCA ET AL: "Comparison of pregnancy rates with two estrus synchronization protocols in Italian Mediterranean Buffalo cows." THERIOGENOLOGY, vol. 60, no. 1, June 2003 (2003-06), pages 125-133, XP002291500 ISSN: 0093-691X

## Description

This invention relates to a method for synchronising ovulation in cattle and a kit of parts for use in synchronising ovulation in cattle.

Pharmacological control of the reproductive cycle of female cattle generally uses hormones to control oestrus and ovulation for the benefit and convenience of the breeder. The administration of such hormones to control the reproductive process in domestic animals such as horses, sheep, pigs, cattle and goats is well known in the art.

Such programs for pharmacological control of the reproductive cycle may be used either to treat pathological conditions in individual animals, or to synchronise ovulation in groups of animals. Reliable systems for reproductive cycle control have been sought by physiologists and veterinarians for many years, since such techniques offer several management advantages.

Their main benefits are synchronisation of a predictable oestrus and ovulation, allowing the use of fixed time artificial insemination (Al) or embryo collection and transfer without spending time on tedious oestrus detection. Some of these programs are additionally able to induce ovulation of non- cycling females, a condition, which is very common during the post -partum period of beef cows or to hasten the onset of cyclicity in virgin heifers. This is very useful as it reduces the calving to conception interval.

Thus synchronisation of a predictable oestrus and ovulation allows batch management of inseminations and results in a predictable time of births. This improves the efficiency of management.

The control of the oestrus cycle is achieved by mimicking the hormonal events occurring during the normal ovarian/oestrus cycle. The development of an ovarian follicle to ovulation in the cycling female cattle is allowed by regression of the corpus luteum (luteolysis) resulting in a decrease in progesterone concentrations. This naturally occurs between day 17 and 18 of the normal cycle. This event may be manipulated artificially and therefore synchronisation follows generally two main approaches: The first approach is the artificial induction of premature luteolysis using luteolytic agents. The second approach of synchronisation is to mimic the presence of a corpus luteum by administration of a progesterone for a number of days followed by abrupt withdrawal.

Various types of schedules for synchronising ovulation making use of either of these two approaches above have been described in the prior art.

Following the first approach, one way is to administer a prostaglandin to initiate corpus luteum regression (luteolysis). Prostaglandin F_{2α} (PGF_{2α}) has been used for a long time in several different ovulation synchronisation protocols. An ovulation synchronisation program that is widely used, especially in dairy heifers, uses the administration of two sequential injections of PGF_{2α} 11 or 14 days apart.

Ovulation is not synchronised very precisely with PGF_{2α} alone and hence fixed time artificial insemination does not result in high fertility. Therefore various attempts have been made to control this variability by suggesting programs that combine the use of prostaglandin with the administration of other hormones such as hCG, a GnRH or an oestradiol. For example US Patent No. US 5,589,457 discloses an ovulation synchronisation program in cattle that uses a sequential injection of a GnRH, a prostaglandin and a second injection of a GnRH.

Following the second approach, several different ovulation synchronisation programs are commercially available where a progesterone is administered by a controlled release device either via the intravaginal route, e.g. PRID® (Progesterone releasing intravaginal device) and CIDR® (controlled internal drug release) or via the subcutaneous route (Crestar® or Synchromate B®). Additional treatments are required to initiate corpus luteum regression e.g. progesterone is combined with e.g. an oestrogen, such as an oestradiol, at the time of progesterone administration, in order to achieve successful synchronisation of oestrus and ovulation. The oestrogen is intended to act as a luteolytic agent and as a synchronizer of follicular turnover. Useful oestradiols for this purpose are e.g. oestradiol -17β, oestradiol benzoate, and oestradiol cypronate or oestradiol valerate.

Various ovulation synchronisation programs comprising a progesterone controlled delivery device in combination with an oestradiol have been used commercially, e.g. Crestar® program (Intervet International), Synchromate B® program (Merial Ltd), CIDR® program (Inter AG) or PRID® program (Ceva Sante Animale). Such programs generally allow fixed time artificial insemination (Al) and result in good fertility in both dairy cows and beef cows.

Xu Z. et al (2000); Journal of Dairy Science 83, 464-470, described synchronisation programs employing an oestradiol by either a) a subsequent administration of progesterone and, at time of artificial insemination, of oestradiol benzoate; or b) a subsequent administration of progesterone, GnRH, PGF_{2α} and, at time of artificial insemination, oestradiol benzoate.

The hormone oestradiol is, however, suspected to both cause and promote breast and prostate cancer and to have potential immunological, neurobiological and genotoxic effects with potentially harmful consequences for human health. It is desired to replace oestradiol with another compound and to retain the good results of fertility and fixed time artificial insemination without employing an oestradiol.

With earlier programs involving the sequential administration of a GnRH, a progesterone and PGF_{2α}, without an oestradiol administration, insemination at detected oestrus had to be done to achieve acceptable fertility (Stevenson J.S. et al (2000); Journal of Animal Science 78, 1747-1758). Alternatively, an additional GnRH injection was done around oestrus to time ovulation for fixed time artificial insemination (Stevenson J.S. et al (1997); Journal of Animal Science 75, 1343-1350, and Thompson K.E. et al (1999); Journal of Animal Science 77, 1823-1832).

Also in other programs without oestradiol involving a progesterone, combined with a GnRH and PGF_{2α}, the synchrony of oestrus induced by treatment was inadequate to fertilise females using fixed time artificial insemination. Therefore artificial insemination after time-consuming oestrus detection had to be done to achieve acceptable fertility (Ryan D.P. et al (1999); Animal Reproduction Science 56, 153-168).

In the current invention a new method for synchronising ovulation in cattle was developed that allows not only fixed time artificial insemination (Al), but also results in good fertility without the involvement of oestradiol in the ovulation synchronisation program.

Therefore the present invention provides a non-therapeutical method of synchronising ovulation in cattle, **characterised in that** the method includes the following subsequent steps:
a) administering to a female cattle a dose of a GnRH and a progesterone delivery device,
b) administering to said animal a dose of a prostaglandin F_{2α}, and
c) removing the progesterone delivery device and administering to said animal a dose of a PMSG.

As it is shown in the experiments, the fertility of female beef- or dairy cattle after fixed time artificial insemination is similar in the groups synchronized by the method according to the invention (without the administration of an oestradiol) compared with the group were a conventional commercial system with an oestradiol was used.

The method according to the invention is a suitable ovulation synchronisation program for female cattle. Cattle are animals of the genus *Bos,* family *Bovidae* including wild species such as the yak, gaur, gayal, banteng, and kouprey, as well as domestic breeds including Asiatic water buffaloes *Bubalus,* African buffaloes *Syncerus,* and American bison *Bison.* Cattle are bred for meat (beef cattle) or milk (dairy cattle). The method according to the invention is generally suitable for all female cattle of different breeds, i.e. for dairy or beef heifers and primiparuos or multiparous dairy or beef cows that are non-pregnant in any stage of cycle.

In carrying out the invention, in the first step of the method an initial dose of a gonadotropin releasing hormone (GnRH) is administered to an open (non - pregnant) female cattle at any stage of an oestrus cycle in order to control follicular turnover. The administration of a GnRH causes growth of a new wave of follicles and assures the presence of a corpus luteum and synchronises the growth of a new follicular wave.

With a GnRH the native GnRH or a synthetic analogue of the native GnRH is meant. The native Gonadotropin-releasing hormone (GnRH) is a decapeptide (pGlu-His-Trp-Ser-Tyr-Gly-Leu-Arg-Pro-Gly-NH₂), which is produced from a precursor polypeptide in hypothalamic neurone.

In a preferred method a synthetic GnRH analogue is used. There are several synthetic analogues of GnRH available, e.g. buserelin, azagly nafarelin, deslorelin, gonadorelin, and fertirelin. Especially preferred are buserelin, a synthetic GnRH analogue composed of 9 amino-acids (Pro-His-Trp-Ser-Tyr-D-Ser-Leu-Arg-Pro-NH-CH₂-CH₃₎ or azaglynafarelin, composed of 10 amino-acids (pGlu-His-Trp-Ser-Tyr-[D-Na(2)]-Leu-Arg-Pro-[aza-Gly]).

The GnRH is administered in an amount sufficient to control ovarian follicle function, e. g., depending on the type of GnRH employed, between 1 and 250 µg per animal, preferably between 5 and 50 µg.

GnRH can be applied in general by all application forms that are known in the art for this purpose. GnRH is preferably administered parenterally, e.g. by intramuscular or subcutaneous injection, typically in the form of long-acting depots.

In addition to the GnRH a progesterone delivery device is administered to the animal. The progesterone delivery device is for practical reasons administered simultaneously or in quick succession to the GnRH administration or in reverse order because in this case the animal must be handled only once and the veterinarian can administer the progesterone delivery device and the GnRH injection within a short time interval. In general the time interval between the administrations can be for the achievement of the positive effects of the current invention extended to 24 hours. Preferably the progesterone delivery device is administered in quick succession to the administration of GnRH or in reverse order.

The administration of a progesterone mimics the corpus luteum function for the duration when the progesterone delivery device is inserted. The gonadotropin release and hence follicular maturation is suppressed until progesterone withdrawal.

The progesterone delivery device comprises progesterone or a synthetic progesterone analogue. In a preferred method according to the invention a synthetic progesterone analogue, (progestagen) like e.g. norgestomet, medroxyprogesterone, norgestrel, levonorgestrel, norethindrone or mesgesterol acetate is used.

Especially preferred is the synthetic progesterone analogue norgestomet. Norgestomet is the INN (international non-proprietary name) of 17α-acetoxy-11β-methyl-19-norpreg-4-ene, 20-dione.

The progesterone delivery device is preferably a controlled release device. A controlled release delivery device is capable of administering a drug over a prolonged or extended period of time. Therefore, it is not necessary to repeatedly e.g. daily administer a progesterone to the animal.

Controlled release devices are the most suitable method of administration of progesterone since withdrawal can then be precisely controlled by device removal. Examples of suitable controlled release devices are the well known intramuscular - or subcutaneous implants or intravaginal inserts.

Implants are objects or materials, such as an alloplastic material or tissue that can be partially or totally inserted or grafted into the body for prosthetic, therapeutic, diagnostic, or experimental purposes.

Progesterone delivery devices for the use in the method according to the current invention are known in the art. For example, the commercial product PRID® (Progesterone releasing intravaginal device) or CIDR® insert (controlled internal drug release) are used presently to deliver a progesterone via the intravaginal route. For the subcutaneous route of administration the implant of the commercial products Crestar® or Synchromate B® are presently used.

PRID® (Ceva Sante Animale) is a specialised form of controlled release device that is inserted and held within the female cattle vagina. PRID® consists of a stainless steel coil covered by a layer of inert silastic in which progesterone is impregnated. The progesterone is released from the elastomer until removal of the device by pulling on the string, which is left protruding from the vulva after coil insertion.

CIDR® cattle insert (Inter AG, Hamilton) is also a controlled release device that is inserted and held within the female cattle vagina. It is a T-shaped insert and is placed into the vagina of female cattle by using a lubricated applicator. The applicator collapses the wings for insertion into the vagina. Expulsion of CIDR® within the vagina causes relaxation of the wings and retention of CIDR® within the vagina by pressure on the vaginal wall. The progesterone is released from the device until removal of the device by pulling on the string, which is left protruding from the vulva after device insertion.

Crestar® (Intervet) and Synchromate® B (Merial) are subcutaneous implants containing norgestomet. The implant is inserted subcutaneously behind the ear during which time the progesterone is absorbed into the blood circulation. The implant is removed by making a small scalpel incision in the skin of the ear over the implant.

The progesterone release device may typically release a progesterone that lead to a blood concentration of progesterone of 1 to 6 ng/ml, preferably to 2 to 5 ng/ml.

Subsequently in step b) of the method according to the current invention a prostaglandin F_{2α} is administered to initiate corpus luteum regression.

The prostaglandin F_{2α} is administered after a suitable period of time that is sufficient for corpora lutea to become sensitive to the luteolytic action of prostaglandins. Such a period of time is normally about 6-9 days after the initial administration of a progesterone delivery device. A satisfactory and preferred standard interval for administration of the PGF_{2α} is seven days after initial administration of a progesterone delivery device.

Suitable compounds are native prostaglandin F_{2α} (PGF_{2α}) and synthetic analogues of prostaglandin F_{2α}. Preferred are synthetic analogues of prostaglandin F_{2α}. The synthetic prostaglandin F_{2α} analogue is preferably selected from the group of cloprostenol, tiaprost, dinoprost, luprostiol, alphaprostol and fenprostalene. Preferred synthetic analogues of prostaglandin F_{2α} are luprostiol and cloprostenol.

The PGF_{2α} will normally be applied at a dose level of 25 mg, which is consistent with the normal use of this hormone to terminate the luteal phase of the oestrus cycle. While this may vary somewhat, normal practice should be to keep the PGF_{2α} within a range of about 20-35 mg. The dosages described above are with respect to native hormones. Synthetic analogues may be used and as will be recognised by those skilled in the art such analogues may be used in smaller amounts owing to their higher activities. PGF_{2α} can be applied to an animal in general by all application forms known in the art. While PGF_{2α} is preferably administered parenterally, e.g. by intramuscular or subcutaneous injection, administration via alternative routes is also possible.

In step c) of the method according to the current invention the progesterone delivery device is removed and a pregnant mare serum gonadotropin (PMSG) is administered. Usually about 1 to 2 days after administration of PGF_{2α}, preferably after 2 days the progesterone delivery device is removed. This removal results in an abrupt withdrawal of progesterone. The removal of the progesterone delivery device from the body can be performed e.g. by removal of the intra-vaginal insert through the vaginal opening or by removal of the ear implant by scalpel incision.

In addition to the removal of the progesterone delivery device a mare serum gonadotropin (PMSG) is administered to the animal. For practical reasons the progesterone delivery device is removed and a PMSG is administered simultaneously or in quick succession or the acts are performed in reverse order because in this case the animal must be handled only once and the veterinarian can perform both acts within a short time interval. In general the time interval between the both acts can be longer. For the achievement of the positive effects of the current invention it can be generally said that the time interval should not exceed 24 hours between both acts. Preferably the PMSG is administered in quick succession to the removal of the progesterone delivery device or in reverse order.

The administration of PMSG will help to achieve ovulation induction in female cattle, which were non-cycling at the beginning of treatment and sustain follicular maturation in all female cattle.

Native pregnant mare serum gonadotropin (PMSG) is a natural glyco-protein consisting of two sub-units (alpha and beta). For use in veterinary medicine native PMSG is extracted from serum of pregnant mares. Alternatively recombinant PMSG can be used. PMSG has follicle stimulating (FSH-like) and luteinising (LH-like) activities. In veterinary medicine PMSG is recommended for the stimulation of ovarian function and activity, improvement of fertility in oestrus-synchronised animals and the induction of super-ovulation.

Commercial PMSG formulations are available as lyophilised preparations in combination with a solvent for intramuscular and subcutaneous use. PMSG can be applied to an animal in general by all application forms known in the art. While PMSG is preferably administered parenterally, e.g. by intramuscular or subcutaneous injection administration via alternative routes is also possible.

Preferably 400 to 600 I.U. of PMSG are administered in the method according to the invention.

As will be understood by those skilled in the art, the significant factors in the reproductive process in cattle can be summarised as follows. For pregnancy to occur, live sperm must be present in the reproductive tract at the time of ovulation. Ovulation occurs about one day after the first signs of oestrus. During natural service, the bull introduces the sperm during the time the animal is showing oestrus.

Successful use of artificial insemination requires that semen be placed in the reproductive tract close to the time of ovulation. Thus, producers watch for signs of oestrus because this is the best indication that an animal is close to ovulation. However, only about 50% of oestrus periods are detected on an average dairy farm.

The present invention can be applied in a manner that completely eliminates oestrus detection in treated female cattle.

After applying the method according to the invention breeding of the animal can be made once between 36 and 60 hours after the progesterone delivery device was removed and PMSG was administered. Alternatively breeding can occur twice between 36 and 60 and 70 to 90 hours after the progesterone delivery device is removed.

The synchronisation procedure of the present invention can be also combined with conventional oestrus detection methods. In this case, the female cattle are bred, once it displays oestrus.

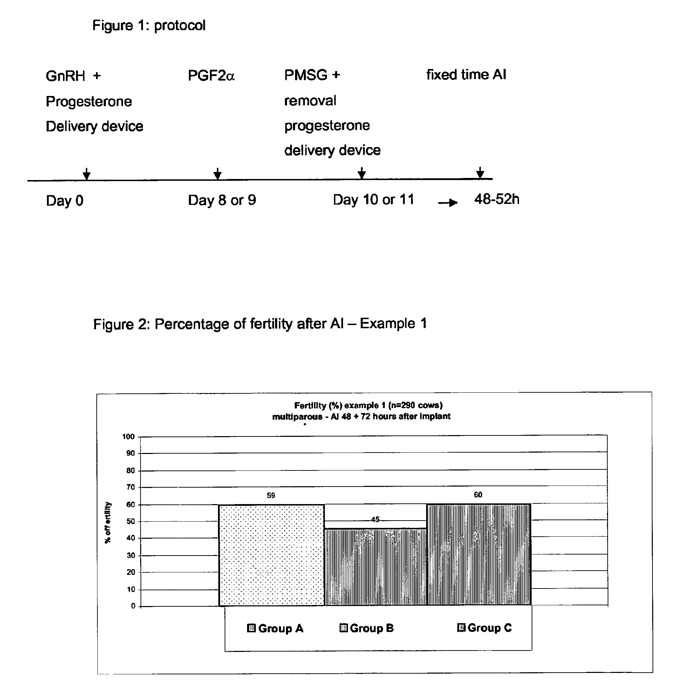

A suitable protocol, illustrating the method according to the invention is shown in the diagram in figure 1.

Another aspect of the invention is a kit of parts for use in synchronising ovulation in cattle **characterised in that** it comprises a dosage form of a GnRH and a progesterone delivery device for initial supply to the female cattle and information means in the form of a label or leaflet suitable to instruct the kit's user that a dosage form of a prostaglandin F_{2α} and a dosage form of a PMSG are to be administered subsequently to said animal.

Preferably the kit of parts comprises additionally a dosage form of a prostaglandin F_{2α} and a dosage form of a PMSG for subsequent supply to said animal.

The kit of parts comprises information means in the form of a label or leaflet suitable to instruct the product's users that
- the GnRH and the progesterone delivery device are to be administered within 24 hours, and that
- the prostaglandin F_{2α} is to be administered 6 to 9 days after the administration of the GnRH, and that
- the progesterone delivery device is to be removed 1 to 2 days after the administration of the prostaglandin F_{2α} and within 24 hours a PMSG is administered.

### Example 1

### Comparison of ovulation synchronisation programs in multiparous beef cows

The aim of this randomised, non-blinded clinical trial was to compare, under field conditions, the safety and efficacy of three injectable solutions associated to a norgestomet implant on fertility and oestrus synchronization of beef cows.

Material and Methods: To multiparous Charolais cows (n=290) at least 60 days post partum a norgestomet implant was administered. At implant insertion (Day 0), additionally one of the following three solutions was intramuscularly (i.m.) injected: Group A norgestomet implant + i.m. injection of 3 mg norgestomet and 3.8 mg oestradiol valerate,
Group B norgestomet implant + i.m. injection of 3 mg norgestomet,
Group C norgestomet implant + i.m. injection of 10.5 µg buserelin

Female cattle of the Groups B and C received one prostaglandin injection (PGF_{2α}) i.m. on day 8 or 9.

The norgestomet implant was removed on day 10 or 11 and an injection of PMSG (i.m 500 or 600 IU) was given simultaneously. Fixed time artificial insemination (48 and 72 hours after implant removal) was used to breed the cows.

Results: The percentage of cows pregnant at fixed time artificial insemination was similar (59% and 60%) in Group A and C respectively whereas it was 45% in the Group B (Figure 2).

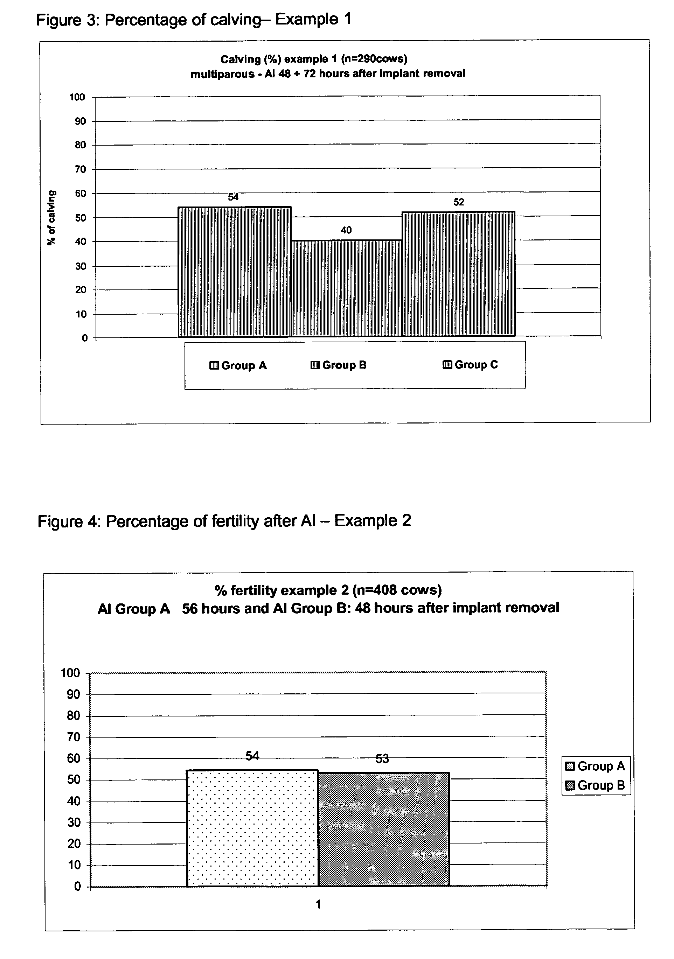

The percentage of calving was similar (54% and 52%) in the Group A and C respectively whereas it was 40% in Group B (Figure 3).

Treatment was the only parameter affecting fertility when logistic regression was used. Fertility in Group A and C was equivalent and numerically higher than in Group B.

From this study, it is concluded that following ovulation synchronisation with a norgestomet implant combined with two different injectable solutions the fertility was similar in Groups A, employing an injectable solution of norgestomet and oestradiol and in Group C employing an injectable solution of buserelin.

### Example 2

### Comparison of ovulation synchronisation programs in heifers, primiparous and multiparous beef cows

The aim of this randomised, non blinded clinical trial is to compare, under field conditions, the efficacy of two injectable solutions associated to a norgestomet implant on oestrus synchronization and fertility of beef cows.

Material and Methods: To heifers, primiparous (at least 60 days post partum) and multiparous (at least 55 days post partum) cows a norgestomet implant was administered.

At implant insertion (Day 0), one of two injectable solutions was intramuscularly (i.m.) injected.
Group A norgestomet implant + i.m. injection of a solution containing 3 mg norgestomet and 3.8 mg oestradiol valerate,
Group B norgestomet implant + i.m. injection of a solution containing 10.5 µg buserelin

In addition, females of group B received a prostaglandin injection (PGF_{2α}) i.m. on day 8 or 7. The implant was removed on day 10 or 9 and an injection of PMSG (i.m. 400 to 600 IU according to parity and farmers' habits) was given simultaneously. Fixed time artificial insemination (56 hours after implant removal for Group A and 48 hours after implant removal for Group B) was used to breed the cows.

Results: The percentage of cows pregnant at fixed time artificial insemination was similar (54% and 56%) in Group A and B (Figure 4). The percentages of heifers and primiparous cows pregnant at fixed time artificial insemination was 47% in the Group B whereas it was 58% in Group B for the multiparous cows.

From this study, it is concluded that following oestrus synchronisation with norgestomet implant combined with two different injectable solutions containing norgestomet and oestradiol valerate (Group A) or buserelin (Group B), the fertility was similar in the Group A with fixed time artificial insemination at 56 hours after implant removal and the Group B with fixed time artificial insemination at 48 hours after implant removal.

## Claims

1. A non-therapeutical method of synchronising ovulation in cattle, **characterised in that** the method includes the following subsequent steps:
a) administering to a female cattle a dose of a gonadotropin releasing hormone and a progesterone delivery device,
b) administering to said animal a dose of a prostaglandin F_{2α,} and
c) removing the progesterone delivery device and administering to said animal a dose of a pregnant mare serum gonadotropin.

2. The method according to claim 1, **characterised in that** in step b) the dose of the prostaglandin F_{2α} is administered 6 to 9 days after administration of the progesterone delivery device, and in step c) the progesterone delivery device is removed 1 to 2 days after the administration of the prostaglandin F_{2α.}.

3. The method according to claims 1 or 2, **characterised in that** in step b) the dose of the prostaglandin F_{2α} is administered 7 days after administration of the progesterone delivery device, and in step c) the progesterone delivery device is removed 2 days after the administration of the prostaglandin F_{2α}.

4. The method according to claims 1 to 3, **characterised in that** the gonadotropin releasing hormone is selected from the group of buserelin, azagly nafarelin, deslorelin, gonadorelin and fertirelin.

5. The method according to claims 1 to 4, **characterised in that** the progesterone delivery device comprises a synthetic progesterone analogue.

6. The method according to claims 1 to 5, **characterised in that** the progesterone delivery device is in the form of a subcutaneous implant.

7. The method according to claims 1 to 6, **characterised in that** the prostaglandin F_{2α} is selected from the group of cloprostenol, dinoprost, luprostiol and fenprostalene.

8. A kit of parts for use in synchronising ovulation in cattle, **characterised in that** it comprises a dosage form of a gonadotropin releasing hormone and a progesterone delivery device for initial supply to a female cattle and a dosage form of a prostaglandin F_{2α} and a dosage form of a pregnant mare serum gonadotropin for subsequent administration to said animal.

9. The kit of parts according to claim 8, **characterised in that** it comprises information means in the form of a label or leaflet suitable to instruct the product's users that
- the GnRH and the progesterone delivery device are to be administered within 24 hours, and that
- the prostaglandin F_{2α} is to be administered 6 to 9 days after the administration of the GnRH, and that
- the progesterone delivery device is to be removed 1 to 2 days after the administration of the prostaglandin F_{2α} and within 24 hours a PMSG is administered.

10. Use of the kit of parts according to claims 8 to 9 for synchronising ovulation in cattle for fixed time artificial insemination.

## Patentansprüche

1. Nichttherapeutisches Verfahren zum Synchronisieren der Ovulation bei Rindern, **dadurch gekennzeichnet, dass** das Verfahren die folgenden aufeinanderfolgenden Schritte beinhaltet:
a) Verabreichen einer Dosis eines gonadotropinfreisetzenden Hormons und einer Progesteronabgabevorrichtung an ein weibliches Rind,
b) Verabreichen einer Dosis eines Prostaglandin F_{2α} an dieses Tier, und
c) Entfernen der Progesteronabgabevorrichtung und Verabreichen einer Dosis eines Pregnant-Mare-Serum-Gonadotropin an dieses Tier.

2. Verfahren nach Anspruch 1, **dadurch gekennzeichnet, dass** in Schritt b) die Dosis des Prostaglandin F_{2α} 6 bis 9 Tage nach der Verabreichung der Progesteronabgabevorrichtung erfolgt und dass in Schritt c) die Progesteronabgabevorrichtung 1 bis 2 Tage nach der Verabreichung des Prostaglandin F_{2α} entfernt wird.

3. Verfahren nach Anspruch 1 oder 2, **dadurch gekennzeichnet, dass** in Schritt b) die Dosis des Prostaglandin F_{2α} 7 Tage nach der Verabreichung der Progesteronabgabevorrichtung erfolgt und dass in Schritt c) die Progesteronabgabevorrichtung 2 Tage nach der Verabreichung des Prostaglandin F_{2α} entfernt wird.

4. Verfahren nach den Ansprüchen 1 bis 3, **dadurch gekennzeichnet, dass** das gonadotropinfreisetzende Hormon aus der Gruppe Buserelin, Azaglynafarelin, Deslorelin, Gonadorelin und Fertirelin stammt.

5. Verfahren nach den Ansprüchen 1 bis 4, **dadurch gekennzeichnet, dass** die Progesteronabgabevorrichtung ein synthetisches Progesteronanalog umfasst.

6. Verfahren nach den Ansprüchen 1 bis 5, **dadurch gekennzeichnet, dass** die Progesteronabgabevorrichtung in Form eines subkutanen Implantats vorliegt.

7. Verfahren nach den Ansprüchen 1 bis 6, **dadurch gekennzeichnet, dass** das Prostaglandin F_{2α} aus der Gruppe Cloprostenol, Dinoprost, Luprostiol und Fenprostalen stammt.

8. Aus Teilen bestehendes Kit für die Verwendung bei der Synchronisierung der Ovulation bei Rindern, **dadurch gekennzeichnet, dass** es eine Darreichungsform eines gonadotropinfreisetzenden Hormons und eine Progesteronabgabevorrichtung für die ursprüngliche Bereitstellung an ein weibliches Rind und eine Darreichungsform eines Prostaglandin F_{2α} und eine Darreichungsform eines Pregnant-Mare-Serum-Gonadotropin für die darauffolgende Verabreichung an dieses Tier umfasst.

9. Aus Teilen bestehendes Kit nach Anspruch 8, **dadurch gekennzeichnet, dass** es Informationsmittel in Form eines Etiketts oder einer Broschüre, das/die geeignet ist, die Verwender des Produkts darüber zu informieren, dass
- das GnRH und die Progesteronabgabevorrichtung innerhalb von 24 Stunden zu verabreichen sind, und dass
- das Prostaglandin F_{2α} 6 bis 9 Tage nach der Verabreichung des GnRH zu verabreichen ist, und dass
- die Progesteronabgabevorrichtung 1 bis 2 Tage nach der Verabreichung des Prostaglandin F_{2α} zu entfernen ist und dass innerhalb von 24 Stunden ein PMSG verabreicht wird,
umfasst.

10. Verwendung des aus Teilen bestehenden Kits nach den Ansprüchen 8 bis 9 für die Synchronisierung der Ovulation bei Rindern bei der terminorientierten künstlichen Besamung.

## Revendications

1. Procédé non thérapeutique de synchronisation de l'ovulation chez les bovins, **caractérisé en ce que** le procédé comprend les étapes subséquentes suivantes :
a) l'administration à un bovin femelle d'une dose de gonadolibérine et d'un dispositif délivrant de la progestérone,
b) l'administration audit animal d'une dose d'une prostaglandine F_{2α} et
c) le retrait du dispositif délivrant de la progestérone et l'administration audit animal d'une dose de gonadotrophine de sérum de jument gravide.

2. Procédé selon la revendication 1, **caractérisé en ce que** dans l'étape b) la dose de la prostaglandine F_{2α} est administrée 6 à 9 jours après administration du dispositif délivrant de la progestérone et dans l'étape c) le dispositif délivrant de la progestérone est retiré 1 à 2 jours après l'administration de la prostaglandine F_{2α}.

3. Procédé selon les revendications 1 ou 2, **caractérisé en ce que** dans l'étape b) la dose de la prostaglandine F_{2α} est administrée 7 jours après administration du dispositif délivrant de la progestérone et dans l'étape c) le dispositif délivrant de la progestérone est retiré 2 jours après l'administration de la prostaglandine F_{2α}.

4. Procédé selon les revendications 1 à 3, **caractérisé en ce que** la gonadolibérine est choisie dans le groupe constitué par la buséréline, l'azaglynafaréline, la desloreline, la gonadoréline et la fertireline.

5. Procédé selon les revendications 1 à 4, **caractérisé en ce que** le dispositif délivrant de la progestérone comprend un analogue synthétique de la progestérone.

6. Procédé selon les revendications 1 à 5, **caractérisé en ce que** le dispositif délivrant de la progestérone est sous la forme d'un implant sous-cutané.

7. Procédé selon les revendications 1 à 6, **caractérisé en ce que** la prostaglandine F_{2α} est choisie dans le groupe constitué par le cloprosténol, le dinoprost, le luprostiol et le fenprostalene.

8. Trousse de composants destinée à être utilisée dans la synchronisation de l'ovulation chez les bovins, **caractérisée en ce qu'**elle comprend une forme posologique d'une gonadolibérine et un dispositif délivrant de la progestérone pour administration initiale à un bovin femelle et une forme posologique d'une prostaglandine F_{2α} et une forme posologique d'une gonadotrophine de jument gravide pour administration subséquente audit animal.

9. Trousse de composants selon la revendication 8, **caractérisée en ce qu'**elle comprend des moyens d'information sous la forme d'une étiquette ou d'une notice pour informer les utilisateurs du produit que
- la GnRH et le dispositif délivrant de la progestérone doivent être administrés dans les 24 heures et que
- la prostaglandine F_{2α} doit être administrée 6 à 9 jours après l'administration de la GnRH et que
- le dispositif délivrant de la progestérone doit être retiré 1 à 2 jours après l'administration de la prostaglandine F_{2α} et dans les 24 heures une PMSG est administrée.

10. Utilisation de la trousse de composants selon les revendications 8 et 9 pour la synchronisation de l'ovulation chez les bovins pour l'insémination artificielle à temps fixe.
